Europäisches Patentamt

**European Patent Office** (11) Publication number: **0 010 527**

Office européen des brevets A1

# EUROPEAN PATENT APPLICATION

(21) Application number: **79830031.5** (51) Int. Cl.³: **A 61 F 1/03**

(22) Date of filing: **24.09.79**

(30) Priority: **06.10.78 IT 8347478**
**04.07.79 IT 8341179**

(43) Date of publication of application:
**30.04.80 Bulletin 80 9**

(84) Designated Contracting States:
**AT CH DE FR GB**

(71) Applicant: **LIMA S.a.s. di Carlo LUALDI**
**Casiacco**
**I-33090 Vito d'Asio (Pordenone)(IT)**

(72) Inventor: **Lualdi, Gabriele**
**Via G.B. Cecchini Loc. Picaron**
**San Daniele del Friuli (Udine)(IT)**

(72) Inventor: **Motta, Antonio**
**deceased**
**Udine(IT)**

(72) Inventor: **Callea, Carlo**
**Via S. Caterina 29**
**Pasian di Prato (Udine)(IT)**

(74) Representative: **Petraz, Gilberto**
**G.L.P. S.a.s. di Gilberto Petraz P.LE Cavedalis 6, 2**
**I-33100 Udine(IT)**

(54) Prosthesis for limbs.

(57) A prosthesis for limbs, suitable for treatment of the knee, hip and other joints by prosthesis and for cooperating with substantially incident (10,111) or substantially coaxial (10,11) bones or else for treatment of bone fractures, whereby said prosthesis comprises in mutual cooperation and coordination at least one axially bored tubular element or sleeve insert (25), which includes a fitted seating (27, 127) and, at its front, means (28) to insert it into the bone (10,11) and perhaps to orient and position it when inserted coaxially with the femur (10) and tibia (11), and at least one prosthesis element (46,47) which can be positioned in relation to said fitted seating (27, 127), whereby auxiliary prosthesis means (50) may also be included and perhaps installed in the hip (111), and whereby positioning and fixture means (31, 58) are advantageously present between the bored tubular element (25) and the prosthesis element (46,47) and cooperate with said fitted seating (27,127), and whereby the sleeve insert (25) too is at least partially inserted in a substantially axial direction into the bone (10,11), and whereby at least part of the sleeve insert (25) cooperates with the intermediate tract of the bone or diaphysis (13).

fig.10

Description of the invention entitled:

"Prosthesis for limbs".

------------------

This invention concerns a prosthesis for limbs, namely a device suitable for replacing joints which have ceased to function.

Said invention concerns particularly a prosthe-

sis for limbs which can be applied not only to the joints of the knee or the joints of the hip but which can also be used in treatment of other joints by prosthesis and in the repair and restoration of said other joints.

Treatment by prosthesis, namely the replacement and repair of joints which are not functioning well, has existed for many years.

The first prosthesis operations for the hip were

carried out in I95I by McKee and thereafter by Charnely; they used a cement as a means for fixture of the osseous part to the actual prosthesis

itself.

rif. ዓዮ
0010527

The cement used was methyl methacrylate and was prepared and used for the first time by Ohorohm in 1901.

The good results obtained were soon known all over the world and so treatment of the hip by prosthesis spread quickly and is appli d nowadays in a very great number of orthopaedic centres.

This discovery has enabled many persons suffering from arthritis of the hip or affected by fractures of the neck of the femur to recover the motory activities of the limbs in question quickly and normally.

The problems visualized are of two types: one type of problem concerns infections, whereas the other type is connected with aseptic detachment of the prosthesis caused by microfractures in the cement or by the ageing of the latter.

Furthermore, there is the problem of removal or replacement of the prosthesis for the hip or knee, some years after it has been applied, owing to the polymerization of the cement, which has been reduced to a slime by the time it reaches the operating table.

The problem of materials has been so fully studied and investigated that nowadays almost the best possible type of material has been found for use in prosthesis, whether said material be a metal or plastic or ceramic, on the basis of examination of the wear properties of said materials and their ability to be tolerated by the human body.

Not many years after treatment of the hip by prosthesis had begun, experiments were also made with treatment of the knee by prosthesis and thereafter of other joints as well.

The first real prosthesis operations for the knee were carried out by Waldius in 1957, and these were followed by prostheses applied by so many other specialists in this field that nowadays there are commercially available about 450 kinds of prosthesis for the knee (Bulletin No.8 of Hawmedica).

So great a number of prostheses for the knee, whether of a hinge type or articulated type or sliding type or two-compartmental type, means that an ideal prosthesis for the delicate knee joint in the human body has still not been realized.

This is so because of the complex anatomical/functional structure of the knee but, even more so, because, to fix the prosthesis thereof or, if desired, of other joints too, it is necessary to use the aforesaid cement, which itself is precisely the painful part of all prostheses and, particularly so, of prostheses of the knee and hip.

A report was made in specialized literature on an experimental study conducted in 1976 regarding a prosthesis of the femur done by removing the head thereof but leaving the great trochanter and a big part of the trochanteric fossa.

This experiment, which was not followed by others, visualized the prosthesis of a hip with a device screwed into the compact tissue of the tract join- ing the epiphysis to the diaphysis.

Such a device involves many drawbacks both during an operation for prosthesis and also thereafter.

A first drawback is the accuracy needed in removing the head of the femur.

A second drawback lies in the fact that the pros- thesis device consists of several elements hinged together and envisages the element constituting the head of the femur as also being partially the lesser trochanter.

A further drawback is the need to make a hole much longer than is strictly necessary both so as to allow the blood to collect without being compressed (so as to avoid the danger of an embolism) and also because of the length required for the element being screwed in.

Yet another drawback lies in the fact that the actual fixture of the threaded edge of the device to the compact tissue takes place in a position too greatly displaced towards the diaphysis, thereby creating concentrated and misaligned strains; this shortcoming leads to a lesser resistance to the stresses which the limb will have to undergo.

Another drawback is the mechanical and non-anatomical solution of the problem.

A drawback also lies in the inability to have a micrometric adjustment of the element being screwed in.

A further drawback is the pocket of compressed and obstructed blood which forms below the element being screwed and between said element and the medullary material.

Another drawback is the possibility that the element thus screwed will become unscrewed.

A further drawback is the possibility that the connecting pin will become loose or will move; if the coupling is made very precisely, such a movement will prove to be an insuperable difficulty during installation of the pin itself.

Another drawback is the possibility that the com- ponents may rotate reciprocally and that the element to reproduce the head of the femur may rotate in relation to the bone under conditions of tensile load.

There is also a prosthesis for the hip whereby the inserted socket within which the head of the femur is lodged is screwed into the bone.

This partial solution to the problem in the form in which it has been realized and envisaged cannot be used for long bones and at the same time raises many problems too as regards anchorage in the hip, as all specialists are well aware.

All these drawbacks and shortcomings which characterize the prostheses existing today have led inventors to investigate and try new and more anatomical solutions which are easier and safer to instal and which involve lesser risks and disadvantages.

The invention set forth here meets the most exacting principles of biomechanics in the field of treatment by prosthesis and leads to the following advantages:

- quick and ready application and positioning;

- solid fixture in the bone without any cement in a position near to the epiphysis;

- a mechanically stable and easily positioned fixture of the components to each other;

rif. ゚ 0010527

- minimal resection of bone;

- the ability to make possible interchanges of the articular portions;

- quick ability to move the limb and an early acceptance of tensile loads after the operation.

Moreover, the invention is suitable for the repair of fractures and for a good outcome thereof.

The invention which comprises these advantages is realized by using a sleeve insert consisting of a hollow tubular element having a lengthwise section like a slightly truncated cone, being substantially circular and advantageously being threaded externally, with possible partial removals of the threads so as to prevent unscrewing and to make self-threading possible, whereby said sleeve insert comprises at least one fitted seating and an axial hole which advantageously passes through it and whereby the fitted seating is able to cooperate with the axial hole.

At a position coinciding with its greatest section said sleeve insert comprises some means to apply the necessary screwing force and to act as a reference point for its positioning.

The conformation of the threads is such as to permit natural development of the bone around the

device after the operation.

According to one variant said perforated hollow tubular element or sleeve insert may have a circular section; according to another variant the outside of the perforated hollow tubular element, whether the latter be axially a truncated cone or a cylinder, can be advantageously formed with lengthwise or circumferential protrusions so as to be of a type which can be inserted by means of compression. According to a further variant said hollow tubular element will have a section consisting of two or more lobes.

The shape of the fitted seating and its position within the sleeve insert determine the use of the sleeve insert itself.

And so we can use the sleeve insert in two distinct ways, namely for prosthesis of the knee or other bones which are substantially coaxial, or else for prosthesis of the hip or other bones which are substantially incident.

Depending on the use to which the invention is to be put, the latter is completed with one or more parts which constitute one integrating and irreplaceable piece together with the sleeve insert itself.

Let us now look at the two specific uses individ-

...arily. In the case of prosthesis of the knee the fitted seating is substantially coaxial with the sleeve insert and may have an advantageously tapered lengthwise section. According to a variant the fitted seating present inside and on the axis of the sleeve insert can also be cylindrical. Another variant is that the fitted seating, whether cylindrical or tapered, comprises lengthwise ridges, for instance, of the type called "knurled edges".

In the case of application to a knee the second element, which constitutes the actual prosthesis itself, tends advantageously to invest a very restricted zone relative to the articular cartilage.

Said second element comprises anchorage means for introduction into the fitted seating of the sleeve insert and, advantageously, peripheral and/or frontal fixture means in the resected bone, whereby the anchorage means to be introduced are advantageously equipped with axial outlets.

Said axial outlets may consist of holes or of peripheral grooves of a lengthwise or spiral type and serve to obviate excessive pressures in the lower part of the sleeve insert, for said lower part is closed when the second element has been fitted.

The prosthesis for the knee is applied by resecting, across the axis of the diaphysis, only the end part of the articular cartilage and perforating the tissue suitably on the same axis as the diaphysis until the compact tissue of the neck joining the epiphysis to the diaphysis has been reached.

Next, the sleeve insert is screwed in or introduced and centres itself in the diaphysis, which has a compact outer tissue that assists this action.

Since the sleeve insert is perforated, when it has been screwed or inserted into its position it does not include chambers below itself to compress the blood and in this way the danger of a fatty embolism or any other type of embolism is obviated.

The sleeve insert can be adjusted micrometically, its position as regards the prosthesis being of no importance in respect of the perfect positioning of the prosthesis itself in relation to the bone.

The next phase is the insertion of the element to anchor the prosthesis in the calibrated seating of the sleeve insert; at the same time the prosthesis is positioned in relation to the joint.

Mutual fixture between the sleeve insert and the prosthesis can be brought about by simple pressure, as takes place in Morse tapered couplings in machine tools. Mutual anchorage can also take place by means

0010527

of expansion.

As said earlier, the element for anchorage of the prosthesis comprises some axially extending peripheral grooves that enable the blood to flow freely from beneath the prosthesis without any compression and without generating any risk of fatty embolism or any other type of embolism.

The anchorage element can bear at its end the prosthesis already formed or can have a seating whereonto the prosthesis is installed. The anchorage element can also comprise frontal anchorage means for the prosthesis in respect of the resected area of the bone.

Where there is a prosthesis of the hip, the fitted seating is positioned advantageously on an axis crossing the axis of the sleeve insert so as to realize an anatomical prosthesis.

The invention tends to avoid a traumatic application of substitutes for the hip joint and tends at the same time to avoid risks of fatty embolisms and also dangers due to a long and substantially immobile stay in hospital.

In cooperation with the sleeve insert and the fitted seating present therein, this invention foresees positionable means which function like the head

0010527

of the femur and which cooperate with socket-type means of a known kind to restore in the hip the seating for a natural lodging of the head of the femur.

According to the invention, the upper part of the femur near the great trochanter is resected along a plane substantially at right angles to the axis of said femur.

After the above has been carried out, an insertion hole is made which cooperates with the intermediate and medullary zone of the upper part of the femur, the axes of which coincide substantially.

Into this hole is screwed or introduced by pressure a hollow tubular piece or sleeve insert which has suitable characteristics.

As the hollow tubular piece or sleeve insert is empty axially, no action to create pressure on the blood takes place during said introduction inasmuch as the blood can flow away freely; in this way the dangers of an embolism of any type are eliminated.

In the case of a prosthesis for the hip the sleeve insert has its fitted seating in an intermediate and incident position, said fitted seating consisting of a crosswise hole inclined in relation to the axis of the sleeve insert.

Said crosswise hole may be smooth or threaded or may include a plurality of lengthwise ridges or grooves.

When the sleeve insert is screwed in or introduced, said hole is caused to lie in an anatomical position coordinated with the axis of the neck of the femur and with the axis of the head of the femur.

By using the orientation notch positioned at the top of the sleeve insert and required for the screwing action also, a device for controlling and directing assembly in an anatomical manner can be positioned in relation to the direction of ascent of the fitted seating.

Said device enables the sleeve insert to be positioned accurately in relation to the head of the femur and makes it possible for this action to be carried out easily and very precisely.

As soon as the sleeve insert has been positioned correctly in relation to the head of the femur and the controlling and directing device has been installed on the sleeve insert itself, the neck of the femur is resected in the proper position and an inclined hole is made which passes through the remainder of the neck of the femur. This inclined hole cooperates with said fitted seating located in

rif. s¹p

0010527

an incident manner and in an intermediate position in the sleeve insert.

Into said fitted seating, which in our example is an inclined hole, an element is screwed or introduced which possesses at one end an anatomical reproduction of the head of the femur and at its other end a rod; said element also advantageously comprises an intermediate supporting surface that is advantageously at right angles to the axis of the rod.

Said rod is able to cooperate with the incident fitted seating located in the sleeve insert and can be at least partially threaded or can include some circumferential or axial channels cooperating or not with at least one threaded part.

The supporting surface has the task of positioning the rod advantageously against the frontal resected zone of the neck of the femur. In this way the bone of the femur cooperates with the mechanical means for their reciprocal fixture and positioning and is reinforced by said means at the same time.

If the rod and the incident seating are threaded, there is a risk of excessive compression on the neck of the femur if there should be any mistake in the setting-up process.

To prevent this it is best that the rod should

0010527

have a threaded terminal zone which cooperates with the peripheral part of the body of the femur so that any mistakes made by the person carrying out the operation will not lead to compression of the neck of the femur, and the outer part of the femur will only be skimmed, this in itself being only a very slight damage which will soon be repaired with the regeneration of the bony tissue.

Owing to this invention traumatic lesions in the femur and hip are almost non-existent, the dangers of an embolism have practically disappeared and a patient can quickly take up a motory position again, seeing that between the bone and the mechanical means an intimate and strong union of a clearly anatomical nature is realized even during the operation itself.

The invention, therefore, consists of a prosthesis for limbs which is suitable for the treatment by prosthesis of the knee, hip and other incident or coaxial joints or else for the treatment of bone fractures and which is characterized by comprising in mutual cooperation and coordination:

- an axially bored tubular element or sleeve insert which includes a fitted seating and, at its front, means to apply inserting force,

- and a prosthesis element which can be positioned
  in said fitted seating,

- whereby there may be some possible auxiliary pros-
  thesis means and whereby means will advantageously
  be included to fix the hollow tubular element to
  the prosthesis element.

Let us now use the attached tables, which show
as examples some prostheses for limbs according to
preferential but not restrictive solutions of the
invention, so as to make more evident the idea of
the solution of the invention itself. The tables are
as follows: -

Fig. I gives a see-through view of a prosthesis for
the knee, as seen from in front and in a partial vertical section.

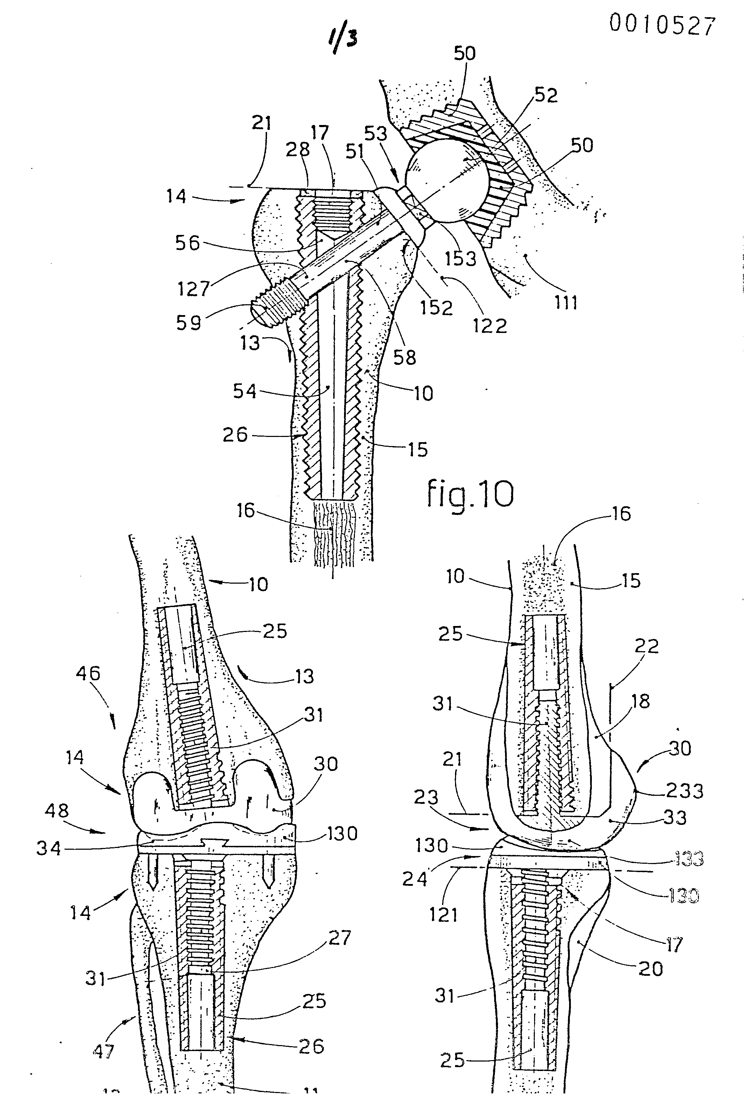

Fig. 2 gives a side view of a vertical section of
a prosthesis for the knee.

Figs. 3 and 4 show an anchorage element with re-
placeable prosthesis means.

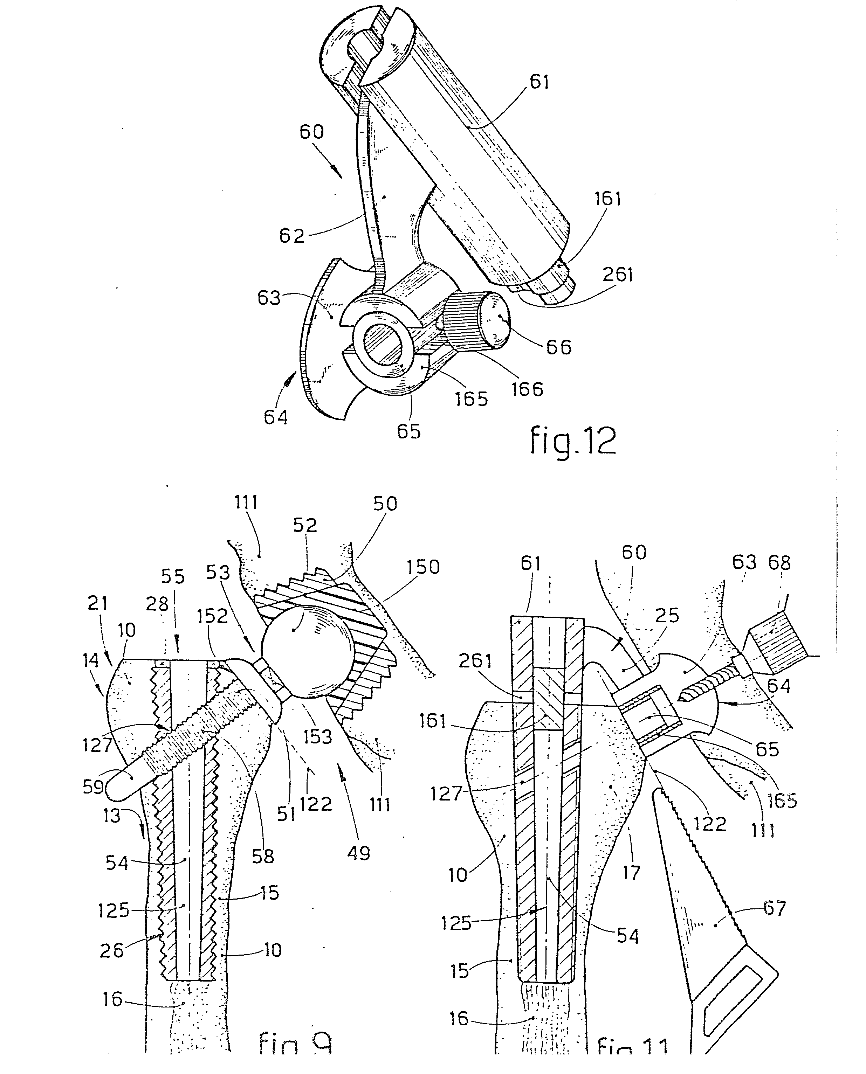

Figs. 5 and 6 show the axially bored sleeve insert.

Figs. 7 and 8 show two variants of the anchorage
means.

Fig. 9 shows a prosthesis for the hip.

Fig.IO shows a variant of Fig. 9.

Fig.II shows an intermediate phase in the applic-

0010527

ation of a prosthesis for the hip.

Fig.I2 shows the device which is equipped for ana-.

tomical control and positioning purposes.

With reference to the figures, the same parts or parts performing the same functions in the tables bear the same reference numbers.

In the figures we have as follows: - I0 is the femur; II is the tibia and III the hip; I2 is the fibula or peroneus; I3 is the intermediate tract of long bone called the "diaphysis"; I4 is the end part of the bone and is called the "epiphysis"; I5 is the compact or cortical bone tissue which composes the wall of the medullary canal I6 of the diaphysis or which composes the inner part of the spongy tissue I7 of the epiphysis I4; I8 is the natural rear central narrowing of the femur in the zone of the epiphysis, whilst I9 is the natural frontal narrowing of the femur in the zone of the epiphysis; 20 is the natural rear narrowing of the tibia in the zone of the epiphysis; 2I is the line of resection of the epiphysis according to the invention and is substantially at right angles to the axis of the femur; I2I is the line of resection at right angles to the axis of the tibia, as realized in the epiphysis of the tibia; 22 is the line of rear resection of the epiphysis of the femur; 122 is the line of resection of the neck of the femur or of the trochanteric fossa and provides the supporting base for the supporting base 152 of the replacement neck 53. for the femur; 23 is generically the femoral element of the prosthesis; 24 is generically the tibial element of the prosthesis; 25 is the hollow sleeve insert and can have a cylindrical or polygonal section or two or more lobes; the sleeve insert can be advantageously cylindrical or tapered lengthwise and can be threaded outside or can possess some lengthwise or circumferential protrusions; in the tables the sleeve insert is shown as being threaded 26 and tapered outside and has a fitted seating 27 in the case of prosthesis of the knee or 127 in the case of prosthesis of the hip; it also has frontal means 28 to apply force for screwing purposes and perhaps for the orientation of the sleeve insert itself.

The fitted seating 27 or 127 can be smooth or knurled axially or circumferentially or can be threaded. The fitted seating 27 is advantageously coaxial with the sleeve insert, whereas the seating 127 is incident.

Thereafter we have the following: - 29 are possible peripheral axial cutaway areas present along

0010527

the axis and perhaps staggered, which can undertake the twofold tasks of screw taps and of means acting to brake any independent unscrewing of the sleeve insert 25; 30 and I30 are generically the anatomical tibial and femoral prosthesis elements; 3I, I3I and 23I are positioning and fixture or anchorage means that cooperate with the fitted seating 27; 32 are outlet channels and can be machined spirally in the anchorage means 3I or can be machined in the form of lengthwise notches or axial holes; 33 is the prosthesis that replaces the end of the femur and has a shape substantially the same as that of the epiphysis itself, being advantageously composed of a terminal body I33 extending with two rear protrusions 233; 34 is the prosthesis that replaces the end of the epiphysis of the tibia; 35 is a possible supporting and positioning base for the actual prosthesis itself; 36 is a possible part of the prosthesis and can be shaped according to 33 or to 34; 37 are the pins for reciprocal fixture of the supporting base 35 to the replaceable prosthesis 36 and of both of them to the front part 2I and I2I of the resected bone; 38 are the radial slits of the anchorage means I3I and cooperate with the terminal bellmouth wherein the axially threaded truncated-cone body 39 is

0010527

lodged and wherein the tensioning screw 40 is screwed; 4I is a possible partially elastic element inserted at the end of the anchorage means 23I; 42 is the fixture rod and has at its end the knob 43, which, as soon as the rod 42 has been thrust down to the bottom of the seating 44, enlarges the elastic element 4I and prevents it from leaving the housing 27; 45 are means for the reciprocal positioning of the elements 35 and 36 and can be of any desired type or shape; 46 is generically the prosthesis for the femoral side of the knee; 47 is generically the prosthesis for the tibial side of the knee; 48 is generically the whole prosthesis for the knee; 49 is generically the prosthesis for the hip; 50 is an element of a substantially known type to replace the acetabulum wherein the head of the femur is lodged in the hip; it is also called the "uncemented cotyloid cavity" and may be of any desired type, namely wholly metallic or wholly plastic, or made of metal 50 combined with plastic I50 or wholly ceramic or made of metal and ceramic, etc.; 5I is the element to replace the head 52 and neck 53 of the femur; 52 is the replacement head for the femur or the anatomical prosthesis element; 53 is the replacement neck for the femur or anatomical prosthesis

BAD ORIGINAL

element and comprises circumferential seatings I53 suitable for means to be applied to regulate the axial position of the element 5I and also comprises the supporting or positioning base I52; 54 is the central bore of the sleeve insert and may have in its upper part 55, where the frontal means 28 are lodged, a threaded seating 56 wherein an appropriate dowel 57 is housed; 55 is the upper part or head of the sleeve insert I25 and can be at right angles to the axis of said sleeve insert or inclined or incident thereto; 58 is the stem of the element 5I and performs the functions of positioning and fixture means; it can be partially threaded, as in Fig. 9 or as in Fig. IO, or can include, perhaps in cooperation with the threaded zones, some circumferential or lengthwise smooth areas, which could also have a helicoidal progress; 59 is the end part of the stem 58 and, as said earlier, can be threaded or not; 60 is generically the means equipped to provide anatomical control and positioning and serves to position and orient the sleeve insert exactly and to ensure accurate removal of the neck of the femur and axial perforation of the neck of the femur itself; 6I is the positioning and orienting body; in our example it comprises both the pin I6I cooperating with the

0010527

axial bore 54 of the sleeve insert I25 and also blade-type protrusions 26I that cooperate with the notches 28 so as to orient and position the means 60 accurately in relation to the direction of ascent and axis of the bore I27; 62 is the connecting fin; 63 is the shaped piece which reproduces partially with its upper part 64 the head of the femur and provides the preferential line for resecting I22 the neck of the femur; 65 is a hollow tubular piece which can be axially stationary or movable, pre-inclined and pre-positioned according to the axis of the bore I27 so that, when the means 60 is positioned above the sleeve insert I25 and the blade-type protrusions I6I are cooperating with the notches 28, the axis of the hollow tubular element 65 lies on the axis of the fitted seating I27; I65 is the stationary hollow tubular piece with a slit, within which the possibly movable tubular piece 65 can run or be fixed as desired; the stationary tubular piece I65 is rigidly connected to and pre-positioned in relation to the positioning body 6I by means of the connecting fin 62; the staggering of the individual parts so as to make the axis of the tubular piece 65 mate with the axis of the seating I27 is a part of normal design techniques; 66 is a possible fixture means

which, by cooperating with the slit 166, serves to fix in the desired position the possible movable tubular piece 65; 67 is indicatively the cutting instrument which serves to resect the neck of the femur in the correct position in relation to the sleeve insert 125 and has the preferential cutting line 122 as a guide; 68 is the drilling means which, cooperating with the guide consisting of the hollow tubular piece 65, perforates the tissue of the neck of the femur in axial cooperation with the fitted seating 127 and thereafter. having passed beyond the fitted seating 127, may perhaps also perforate the compact tissue lying beyond the sleeve insert 125; the drilling instrument 68 obviously has an outer diameter that cooperates with the seating 127 and with the dimensions of the stem 58.

As said earlier, the fitted seatings 27 and 127 can be tapered or cylindrical, smooth, knurled or threaded, axially or circumferentially, depending on the use to which they are to be put.

If the fitted seatings 27 and 127 are tapered, the anchorage means 31 and 58 will be tapered and will behave reciprocally like a tapered mechanical coupling of the Morse taper type, for instance.

If the fitted seating 27 is cylindrical, the an-

0010527

chorage means I3I and 23I can be of diverse types; figures 7 and 8 give examples of two possible types.

Fig. 7 shows a type wherein the effect of the screw 40, which presses with its head into an appropriate seating in the element 35, causes the truncated cone body 39 to be able to move axially, thus provoking an expansion of the wings of the anchorage means, said wings then anchoring themselves to the walls of the fitted seating 27.

Fig. 8, on the other hand, shows a rod 42 which has at its end the knob 43; when the rod 42 is pushed as far as it can go into the seating 44, the knob arrives and cooperates with the at least partially elastic element 4I, causing it to expand and to anchor itself in a part of the fitted seating 27 or at the end thereof.

Let us now see the method of functioning in the case of a prosthesis for the knee (Figs. I to 8 inclusive). When resections have been made along the planes 2I, 22 and I2I, the appropriate axial holes are then made in the femur IO and/or in the tibia II. These holes should be advantageously centred in the central zone of the narrowing of the epiphysis of the femur IO and of the tibia II.

This enables the sleeve insert, when being screwed in, to centre itself when it approaches the tract
of the diaphysis of the long bones.

Owing to the cutaway portions 29 the sleeve insert
25 behaves like a thread-forming element and at the
same time brings about the reciprocal anchorage
which prevents it from becoming unscrewed.

The fact that the sleeve insert 25 has an axial
bore enables the blood to flow freely without the
creation of counterpressures and without the consequent dangers of fatty embolisms or embolisms of
any other type.

As the sleeve insert 25 is relatively long, it
is able to cooperate with the bone and creates
thereon a very light specific load in the zone of
mutual contact and anchorage. At the same time the
anatomical position of the sleeve insert 25 in the
bone is such that moments of forces unlike the normal ones existing in a healthy bone are not created.

When the sleeve insert 25 has been positioned,
the means 3I (or I3I or 23I) are then positioned in
the seating 27.

Even during the positioning of the means 3I, I3I
or 23I in the seating 27 there is no danger of creating any pressure on the mass of blood flowing from
the bone; this is so because of the presence of the

outlet channels 32, which enable the blood to drain away independently.

Even if the prosthesis element 30 does not come into complete contact with the resected surfaces at once, it is not very important since the bone recreates itself and arranges independently to fill the empty spaces within a matter of some days.

The presence of the rear bodies 233 prevents the prosthesis from rotating out of its position; this task is also fulfilled by the pins 37, which are driven into the front part of the resection 2I - I2I of the epiphysis.

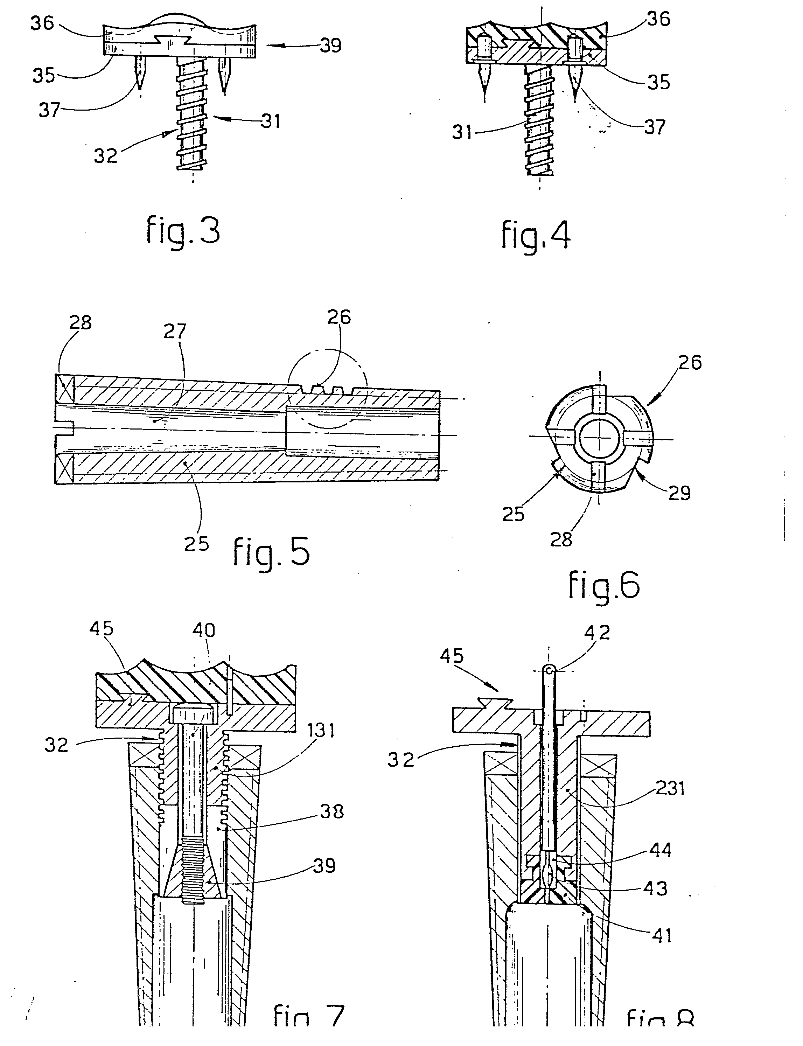

If so wished, the prosthesis can be replaced as shown in Figs. 3 and 4. In this case reciprocal positioning means 45 and means for stable reciprocal fixture 37 will be provided.

Let us now see the method of functioning in the case of a prosthesis for the hip (Figs. 9 to I2 inclusive). After the upper part of the femur IO has been removed along a plane substantially at right angles to the axis of the femur itself IO and near to the great trochanter, an axial hole may be made, but is not necessarily made, in the spongy tissue and in the medulla on the axis of the femur itself. This removal of tissue can be carried out with an

appropriate instrument, which is not shown here as it is not relevant to the purposes of the discovery.

In the hole thus made the sleeve insert I25 is screwed or introduced with a suitable screwing or thrusting means and is positioned in relation to the head 52 of the femur IO by means of the device 60.

When the sleeve insert I25 has been positioned, the neck of the femur is resected, as said earlier, with an appropriate instrument 67 along the cutting line I22 and a hole is made on the same axis as the fitted seating I27. The element 5I is screwed or thrust into the hole thus made until the supporting base I52 mates with and rests against the surface I22 obtained by the resection of the neck of the femur. The element 5I is screwed or thrust, depending on whether the fitted seating I27 is threaded or not. If the end 59 of the stem 58 is threaded, the element 5I is thrust through the fitted seating I27 and is then screwed onto the outer bone of the femur IO. Even if this action is not carried out very accurately, the force exerted by the threads against the outer bone is not such as to impare the remainder of the tract of the neck of the femur in a dangerous manner.

We have described here some variants of the idea of the solution as applied to treatment of the knee and hip by prosthesis, but other variants are possible for a specialist in this field. Thus it is possible to vary the sizes and proportions and to apply the idea of the solution to treatment of the elbow and ankle, etc. by prosthesis and, to be more precise, to other joints; the idea of the solution can be applied as a whole or in part to treatment of broken bones; it is possible to use any material which may be useful for the purpose and advantageous as regards duration or compatibility with the human body; it is possible, for instance, to use polythene, titanium, an alloy based on titanium, ceramics, very pure sintered corundum, etc.; the surface in contact with smooth bone can be made with suitable holes or ridges suitable for enhancing the anchorage and/or the growth of the bone; it is possible to use any type of screw threads and to vary the conformation of the prosthesis on the basis of biomechanical experiments; it is possible to foresee the sleeve insert as being knurled outside or having circumferential ridges; the seatings could be knurled, etc.; these and perhaps other variants are possible within the scope of the idea of the solution.

0010527

# CLAIMS

I.  Prosthesis for limbs, suitable for treatment of the knee, hip and other joints by prosthesis and for cooperating with substantially incident (IO-III) or substantially coaxial (IO-II) bones or else for the treatment of bone fractures, characterized by the fact that it comprises in reciprocal cooperation and coordination:

- at least one axially bored (54) tubular element or sleeve insert (25-I25) which includes a fitted seating (27-I27) and, at its front, means (28) to insert it into the bone (IO-II) and perhaps to orient and position it,

- and at least one prosthesis element (46-47-49) which can be positioned in relation to said fitted seating (27-I27),

- whereby auxiliary prosthesis means (50) may also be included,

- and whereby positioning and fixture means (3I-I3I -23I-58) are advantageously present between the bored tubular element (25-I25) and the prosthesis element (46-47-49) and cooperate with said fitted seating (27-I27),

- and whereby the sleeve insert (25-I25) is at least partially inserted in a substantially axial direct-

0010527

ion into the bone (IO-II).and whereby at least part of said sleeve insert (25-I25) cooperates with the intermediate tract of the bone or diaphysis (I3).

2. Prosthesis for limbs for treatment by prosthesis, as in Claim I, characterized by the fact that the hollow tubular element or sleeve insert (25-I25)has its outer surface equipped (26) to enhance its an- chorage to the bone (IO-II) and has any desired crosswise section, whereby at the front of said sleeve insert (25-I25) there are setting and positioning means (28) and whereby the sleeve insert (25-I25) is at least axially perforated (54) and has a lengthwise section which is advantageously tapered.

3. Prosthesis for limbs for treatment of the knee or other substantially coaxial joints by prosthesis, as in Claims I and 2, characterized by the fact that the hollow tubular element or sleeve insert (25) has the fitted seating (27) substantially on the same axis as the axial hole (54) passing advantageously therethrough.

4. Prosthesis for limbs for treatment of the knee or other substantially coaxial joints by prosthesis, as in Claims I and 3, characterized by the fact that

.the prosthesis elements (46-47) comprise in reciprocal cooperation and coordination:

— anatomical prosthesis elements (30-I30),

— and positioning and fixture means (3I-I3I-23I) which cooperate with the fitted seating (27), whereby the positioning and fixture means (3I-I3I-23I) include outlet channels (32).

5. Prosthesis for limbs for treatment of the knee or other substantially coaxial joints by prosthesis, as in Claim 4, characterized by the fact that the anatomical prosthesis elements (30-I30) are at least partially replaceable (36).

6. Prosthesis for limbs for treatment of the hip and other incident joints by prosthesis, as in Claims I and 2, characterized by the fact that the hollow tubular element or sleeve insert (I25) has the fitted seating (I27) incident in relation to said sleeve insert itself and in relation to the axial hole (54) passing advantageously therethrough.

7. Prosthesis for limbs for treatment of the hip and other incident joints by prosthesis, wherein the prosthesis cooperates with the cotyloid cavity (50-I50) present in the hip (III), as in Claims I and 6, characterized by the fact that the prosthesis element (49) comprises in reciprocal cooperation and coordination:

- anatomical prosthesis elements (52-53)

- and positioning means (I52) and positioning and fixture means (58) which cooperate with the fitted seating (I27),

  whereby the anchorage means (58) can be at least partially threaded.

8.    Prosthesis for limbs for treatment of the knee

  or other substantially coaxial joints, as in Claims

  6 and 7, characterized by the fact that to obtain

  in the femur (IO) the supporting surface (I22) and

  the hole for the introduction of positioning and fixture means (58) a device (60) to provide anatom-

  ical positioning and control is employed which coop-

  erates with the top part (55) of the sleeve insert (I25) and with the frontal means (28) thereof.

fig.10

rif. glp.1-1060

0010527

fig.3

fig.4

fig.5

fig.6

fig.7

fig.8

fig.12

fig.9

fig.11

| Category | Citation of document with indication, where appropriate, of relevant passages | Relevant to claim |
|---|---|---|
| | **DOCUMENTS CONSIDERED TO BE RELEVANT** | |
| A | DE – B1 – 2 338 136 (GEBR. SULZER AG) | |
| | -- | |
| A | DE – A1 – 2 610 922 (SANITÄTSHAUS SCHÜTT & GRUNDEI) | |
| | -- | |
| A | DE – A1 – 2 543 911 (R.A. ELSON) | |
| | -- | |
| A | DE – A1 – 2 558 446 (MAHAY & CIE) | |
| | -- | |
| A | FR – A2 – 2 366 005 (MAHAY & CIE) | |
| | ---- | |

**CLASSIFICATION OF THE APPLICATION (Int. Cl.3)**

A 61 F    1/03

**TECHNICAL FIELDS SEARCHED (Int.Cl.3)**

A 61 F    1/00

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| Berlin | 25-01-1980 | KLITSCH |

EPO Form 1503.1   06.78